Europäisches Patentamt

European Patent Office

Office européen des brevets

(19)

(11) Veröffentlichungsnummer: **0 298 954**
**A1**

## (12) EUROPÄISCHE PATENTANMELDUNG

(21) Anmeldenummer: 88890182.4

(22) Anmeldetag: 08.07.88

(51) Int. Cl.⁴: **C 12 N 11/14**
C 12 N 11/18, A 23 L 2/34

(30) Priorität: 08.07.87 AT 1721/87

(43) Veröffentlichungstag der Anmeldung:
**11.01.89 Patentblatt 89/02**

(84) Benannte Vertragsstaaten:
**AT BE CH DE FR GB IT LI LU NL SE**

(71) Anmelder: **MAGINDAG Steirische Magnesit-Industrie Aktiengesellschaft**
**Fleschgasse 34**
**A-1130 Wien (AT)**

(72) Erfinder: **Birkner, Friedrich, Dr.**
**A-3384 Gross-sierning 33 (AT)**

**Pittner, Fritz, Doz. Dr.**
**Khekgasse 40-42**
**A-1230 Wien (AT)**

**Schalkhammer, Thomas**
**Lechthalergasse 11**
**A-1235 Wien (AT)**

**Turecek, Peter**
**Heiligenstädter Strasse 115/35**
**A-1190 Wien (AT)**

(74) Vertreter: **Kretschmer, Adolf, Dipl.-Ing. et al**
**Patentanwälte Dipl.Ing. A. Kretschmer Dr. Thomas M.**
**Haffner Schottengasse 3a**
**A-1014 Wien (AT)**

(54) **Enzymimmobilisat zur Behandlung von Frucht- und Gemüsesäften sowie Verfahren zu seiner Herstellung.**

(57) Bei einem Enzymimmobilisat zur Behandlung von Frucht- und Gemüsesäften mit einem inerten Träger wie Kieselgel oder Kieselerde werden am Träger, der insbesondere aus trockenem Silicat besteht, Amylasen und Pektinasen gemeinsam immobilisiert.

EP 0 298 954 A1

Bundesdruckerei Berlin

## Beschreibung

### Enzymimmobilisat zur Behandlung von Frucht- und Gemüsesäften sowie Verfahren zu seiner Herstellung

Die Erfindung bezieht sich auf ein Enzymimmobilisat zur Behandlung von Frucht- und Gemüsesäften mit einem inerten Träger wie Kieselgel oder Kieselerde, sowie auf ein Verfahren zur Herstellung eines solchen Enzymimmobilisates.

Für die Herstellung von Enzymimmobilisaten sind verschiedene Verfahren bekanntgeworden, wobei in der Regel eine Adsorption einer Enzymlösung an getrockneten Silicaten als Träger vorgenommen wurde. Als silicatischer Träger kann beispielsweise Gulsenit Verwendung finden. Nach der Adsorption der Enzyme kann eine Ausbildung eines durchgehenden Filmes bzw. einer Hülle durch Quervernetzung mit einem Aldehyd vorgenommen werden. Es ist weiters bekannt, Enzymimmobilisate dadurch herzustellen, daß Kieselgel oder Kieselerde bzw. silicatisches Trägermaterial zunächst einer Modifikation durch Behandlung mit Aminoalkylsilanen und Glutardialdehyd unterworfen wird, wobei das erhaltene Derivat für die kovalente Bindung von Enzymen an Trägern geeignet ist. Weiters sind Verfahren bekannt, bei welchen beispielsweise Serumalbumine zur Immobilisierung herangezogen werden. Auch Pyridinogele sind als Enzymimmobilisat bekannt.

Die Herstellung derartiger Enzymimmobilisate für die Behandlung von Frucht- und Gemüsesäften ist insbesondere mit Rücksicht auf die Tatsache bedeutend, daß derartige Enzymimmobilisate mehrfach verwendet werden können und für eine derartige mehrfache Verwendung regeneriert werden können. Weiters ist insbesondere bei der enzymatischen Behandlung mit Pektinasen die Immobilisierung am Träger deshalb besonders vorteilhaft, weil das erhaltene Produkt weitgehend enzymfrei bleibt. Pektinasen sind in der Regel Schimmelpilzpräparate, welche Toxine enthalten können. Durch die Immobilisierung derartiger Enzyme am Träger kann das erhaltene Produkt von Enzymen und damit auch von Toxinen weitgehend freigehalten werden.

Die Behandlung von Frucht- und Gemüsesäften mit Amylasen und Pektinasen ist prinzipiell bekannter Stand der Technik. Aus der GB-A- 1 368 854 ist es bekannt, Naringinase alleine zur Entbitterung von Fruchtsäften einzusetzen. Die EP-A-17 115 zeigt und beschreibt enzymatische aktive Präparate, welche in Kieselgel eingebracht bzw. eingebettet werden, nicht aber an der Oberfläche eines Trägers immobilisiert sind.

Aus der US-A-4 506 015 ist ein mehrschichtiges Immobilisat bekanntgeworden, wobei die zweite Schichte kovalent an der ersten Schichte gebunden ist. Die kovalente Bindung wird mit Hilfe von Aminosilanen erreicht und die zweite Schichte deckt die erste Schichte ab, so daß die erste Schichte genaugenommen als eingebetten zu bezeichnen ist.

Die Erfindung zielt nun darauf ab ein Enzymimmobilisat der eingangs genannten Art zu schaffen, welches bei der Behandlung von Frucht- und Gemüsesäften eine raschere und effizientere Umsetzung bzw. Klärung von Frucht- und Gemüsesäften erlaubt. Zur Lösung dieser Aufgabe besteht das erfindungsgemäße Enzymimmobilisat im wesentlichen darin, daß am Träger Amylasen und Pektinasen gemeinsam immobilisiert sind. Überraschender Weise hat sich gezeigt, daß ein derartiges Enzymmischimmobilisat eine im Vergleich zu den einzelnen Enzymen in konsekutiver Anwendung wesentlich raschere Umsetzung und Klärung von Frucht- und Gemüsesäften ermöglicht. Während die konsekutive Anwendung von Amylasen, Pektinasen und gegebenenfalls Naringinase einen erheblichen Zeitaufwand für die Umsetzung darstellt und bei konventioneller Anwendung zum Verbleib von Enzymen im Produkt führt, hat sich überraschender Weise gezeigt, daß auch bei kombinierter Anwendung von lediglich Amylase oder Pektinase oder Naringinase enthaltenden Immobilisaten eine längere Zeit für die Umsetzung erforderlich ist, als mit einem Enzymmischimmobilisat, bei welchem eine Mehrzahl derartiger Enzyme gleichzeitig am gleichen Träger immobilisiert sind. Wesentlich für ein derartiges Mischimmobilisat gemäß der Erfindung ist hiebei, daß Amylasen und Pektinasen keineswegs in einen Träger eingebettet werden und aus einem derartigen Träger ausgewaschen werden können, sondern, daß sie vielmehr an der Oberfläche eines derartigen Trägers, d.h. am Träger immobilisiert sind. Der bekannte Synergismus von Pektinasen und Amylasen allein bringt noch nicht den erfindungsgemäßen Effekt, welcher offensichtlich erst dann eintritt, wenn eine vorgegebene Vicinität und eine bestimmte sterische Konfiguration, wie sie durch Anordnung am Träger gegeben ist, erhalten werden kann. Die Inklusion oder Einbettung von Pektinasen ist insbesondere im Hinblick darauf wenig sinnvoll, da ja Makromoleküle nicht in das Innere eines Trägers eindringen können, in welchem derartige Pektinasen eingebettet sind. Die Makromoleküle in Frucht- und Gemüsesäften können keineswegs in Poren des Trägers eindringen, so daß der Effekt der Inklusion von Pektinasen in keiner Weise demjenigen Effekt entspricht, wie durch Vicinität von Pektinasen und Amylasen an der Oberfläche eines Trägers erzielt werden kann.

Die Inklusion oder Anordnung im Träger kann nun theoretisch zu Auswaschungen führen, wobei derartige Auswaschungen offensichtlich immer dann unvermeidbar sind, wenn tatsächlich eine Konfiguration erzielt werden soll, bei welcher Makromoleküle auch mit den im Träger eingebetteten Pektinasen in Berührung kommen sollen. Eine derartige Struktur setzt nämlich eine relativ grobporige Trägerstruktur voraus und bei derartigen grobporigen Strukturen besteht naturgemäß die Gefahr der Auswaschung in besonderem Maße. Mit Vorteil wird für die Behandlung von Frucht- und Gemüsesäften zusätzlich zu Amylasen und Pektinasen auch Naringinase am Träger immobili siert, wobei sich auch hier ein überraschender synergistischer Effekt gezeigt hat.

Die erfindungsgemäßen Enzymimmobilisate enthalten mit Vorteil pro Gramm trockenen Silicates Enzymmischungen mit 150U - 270U α -Amylase, 5000U - 20000U Pektinase und 4U - 40U Naringinase, wobei ein U bzw. eine Einheit je nach dem gewählten Enzym einer bestimmten Aktivitätsdefinition unterworfen ist. Im einzelnen gelten für ein U jeweils die folgenden Definitionen:

α-Amylase (E.C.3.2.1.1.):

1U α-Amylase ist jene Menge Enzym, die 1 Mikromol Maltose pro Minute bei 25°C und pH 4,3 spaltet.

"Pektinase" enthält folgende Enzyme:

Pektinesterase (E.C.3.1.1.11)

Pektatlyase (E.C.4.2.2.2)

Pektinlyase (E.C.4.2.2.10)

Polygalakturonase (E.C.3.2.1.15)

1U "Pektinase" ist jene Menge Enzym, die 33,3 ml eines Standardsaftes (0,04 g Pektin/l00 ml mit einem Veresterungsgrad von 95 % und pH 3,5) bei 55°C in 2 Stunden depektinisiert.

Endpunktsbestimmung: 2 Teile Reagenz (99 Teile Ethanol 96 %, 1 Teil Salzsäure konz.) geben mit 1 Teil Standardsaft in 4 Minuten keine Fällung des Pektin mehr.

Naringinase enthält folgende Enzymaktivitäten:

α-L-Rhamnosidase (E.C.3.2.1.40)

β-D-Glucosidase (E.C.3.2.1.21)

1U "Naringinase" setzt 1,0 Mikromol reduzierende Zucker (gemessen als Glucose) aus Naringin pro Minute bei 40°C und pH 4,0 frei.

Bevorzugt sind Amylase und Pektinase im Einheiten-Verhältnis von 1 : 30 bis 1 : 75, sowie vorzugsweise Naringinase und Amylase im Einheiten-Verhältnis von 1 : 40 bis 1 : 6 am Träger immobilisiert.

Für die Herstellung eines derartigen Enzymimmobilisates wird bevorzugt das Verfahren so durchgeführt, daß die Enzyme und der Träger, insbesondere trockenes Silicat, in einem Lösungsmittel, wie Wasser, suspendiert und gerührt werden und daß nach Durchmischung des Trägers mit den Enzymen Glutardialdehyd unter weiterem Rühren zugesetzt wird, worauf mit Wasser proteinfrei und aldehydfrei gewaschen wird.

Die Erfindung wird nachfolgend an Hand von Ausführungsbeispielen näher erläutert.

Beispiel 1:

1 kg trockenes Silicat (Gulsenit oder ähnliches) wird mit einer Mischung von 0,21 x 10⁶U α-Amylase und 12,5 x 10⁶U Pektinase in 2,8 l Wasser versetzt und 30 Minuten bei Raumtemperatur mit ca. 300 U min⁻¹ gerührt, wodurch eine gute Durchmischung erzielt wurde. Dann wurden 200 ml einer 25 %igen wässrigen Glutardialdehydlösung zugegeben und 20 Stunden bei 20°C weitergeführt. Die Immobilisierung wurde durch Vernetzung der Proteine an der Trägeroberfläche erreicht.

Anschließend wurde mit Wasser protein- und aldehydfrei gewaschen, wobei als Nachweis im Waschwasser für Protein die Ninhydrinreaktion und für Aldehyd die Reaktion mit 2,4-Dinitrophenylhydrazin verwendet wurde. Das wasserfeuchte Immobilisat wurde bei 4°C gelagert und war mehrere Monate haltbar.

Die Behandlung von Obst- und Gemüsesäften erfolgte in einem thermostatisierbaren Rührtankreaktor.

Als Testsubstrate wurden verwendet:
a) Rohapfelsäfte
b) Rohapfelsaft + 1 % löslicher Stärke + 0,5 % Apfelpektin
Acetatpuffer pH4, 1M + 1 % löslicher Stärke + 0,5 % Apfelpektin

Der Reaktionsverlauf wurde mittels folgender Methoden durch diskontinuierliche Messung mit Probenentnahme verfolgt:

Stärkeabbau: Iod-Stärke Reaktion;
Glucosenachweis enzymatisch mit Glucosedehydrogenase.

Pektinabbau: Fällungstest mit salzsaurem Ethanol, Rhamnosenachweis enzymatisch mit Rhamnosedehydrogenase.

Gesamtpolysaccharide: dünnschichtchromatographischer Nachweis von Zuckern und oligomeren Abbauprodukten der polymeren Substrate.

Je 1 l der Testsubstrate wurde mit 50 g des Mischimmobilisates, dessen Herstellung oben beschrieben wurde, bei 50°C unter Rühren inkubiert. Ein quantitativer Abbau aller Substrate war unter den angegebenen Bedingungen in 5 Stunden möglich. Das Immobilisat war ohne Reaktivierung im selben Reaktor bis zu 10 mal wiederverwendbar, wobei nach Absetzen des Immobilisates nach Abschalten der Rührung die überstehende, abgebaute Lösung abgesaugt wurde.

Beispiel 2:

Unter den selben Bedingungen wie im Beispiel 1 wurde ein Immobilisat hergestellt und mit den verschiedenen Substratlösungen wie oben inkubiert. Die Tests und Verfahrensweisen waren analog zu Beispiel 1.

Die Herstellung des Immobilisates erfolgte jedoch unter Einsatz einer Mischung von 0,022 x 10⁶U Naringinase, 0,21 x 10⁶U α-Amylase und 12,5 x 10⁶U Pektinase. Ein solches Immobilisat war in der Lage die eingesetzten Substrate in längstens 3 Stunden bei 50°C abzubauen.

Als Vergleichsbeispiel wurden Monoenzymimmobilisate von α-Amylase und Pektinase hergestellt. Die Herstellung und die Anwendungsbedingungen wurden analog den Bedingungen in Beispiel 1 gewählt und es wurde 1 kg trockenes Silicat 0,21 x 10⁶U α-Amylase und 2,8 l Wasser versetzt und 30 Minuten bei Raumtemperatur gerührt. Dann wurde mit 200 ml 25 % wässriger Glutardialdehydlösung versetzt und 20 Stunden bei 20°C weitergerührt, und anschließend gewaschen. Ebenso wurde 1 kg trockenes Silicat mit 12,5 x 10⁶U Pektinase versetzt und ein entsprechendes Monoenzymimmobilisat hergestellt.

25 g des Amylaseimmobilisates wurden mit 25 g des Pektinaseimmobilisates vermischt (jeweils Feuchtgewicht). Mit diesem Gemisch wurde je 1 l der Testsubstrate (siehe Bsp.1) bei 50°C unter Rühren inkubiert. Der quantitative Abbau aller Substrate benötigte 7 Stunden. Die Immobilisate waren ebenfalls ohne Reaktivierung bis zu 10 mal wiederverwendbar.

Aus dem Vergleich mit dem Zeitbedarf der Enzymmischimmobilisate gemäß Beispiel 1 und 2 ergibt sich, daß das Gemisch der Monoenzymimmobilisate eine wesentlich längere Zeit für den quantitativen Abbau benötigt. Die Enzymmischimmobilisate waren im Bezug auf den Zeitbedarf für den quantitativen Abbau den entsprechenden Monoenzymimmobilisaten klar überlegen. Monoenzympräparate eignen sich aber selbstverständlich ohne weiteres zur quantitativen Depektinisierung von nur pektinhältigen Lösungen, sofern ein Pektinimmobilisat eingesetzt wird bzw. von reinen Stärkelösungen sofern ein Amylaseimmobilisat eingesetzt wird.

Der synergistische Effekt ist vermutlich darauf zurückzuführen, daß gemeinsam mit dem depolymerisierenden Effekt der Pektinasen ein beschleunigter Angriff der Amylasen zur rascheren Zuckerumwandlung bzw. -umsetzung führt.

Enzympräparationen bzw. Enzymmischimmobilisate, welche je Gramm trockenen Silicats, 150U bis 270U $\alpha$-Amylase, 5000U bis 20000U Pektinase und 4U bis 40U Naringinase enthielten, konnten in 2 bis 10 ml Wasser beliebig gemischt werden.

## Patentansprüche

1. Enzymimmobilisat zur Behandlung von Frucht- und Gemüsesäften mit einem inerten Träger wie Kieselgel oder Kieselerde, dadurch gekennzeichnet, daß am Träger Amylasen und Pektinasen gemeinsam immobilisiert sind.

2. Enzymimmobilisat nach Anspruch 1, dadurch gekennzeichnet, daß zusätzlich Naringinase am Träger immobilisiert ist.

3. Enzymimmobilisat nach Anspruch 1 oder 2, dadurch gekennzeichnet, daß pro Gramm trockenen Silicates Enzymmischungen mit 150U - 270U $\alpha$-Amylase, 5000U - 20000U Pektinase und 4U - 40U Naringinase immobilisiert sind.

4. Enzymimmobilisat nach Anspruch 1, 2 oder 3, dadurch gekennzeichnet, daß Amylase und Pektinase im Einheiten-Verhältnis von 1 : 30 bis 1 : 75 am Träger immobilisiert sind.

5. Enzymimmobilisat nach einem der Ansprüche 1 bis 4, dadurch gekennzeichnet, daß Naringinase und Amylase im Einheiten-Verhältnis von 1 : 40 bis 1 : 6 am Träger immobilisiert sind.

6. Verfahren zur Herstellung von Enzymimmobilisaten nach einem der Ansprüche 1 bis 4, dadurch gekennzeichnet, daß die Enzyme und der Träger, insbesondere trockenes Silicat, in einem Lösungsmittel, wie Wasser, suspendiert und gerührt werden und daß nach Durchmischung des Trägers mit den Enzymen Glutardialdehyd unter weiterem Rühren zugesetzt wird, worauf mit Wasser proteinfrei und aldehydfrei gewaschen wird.

<table>
<tr><th colspan="2" style="text-align:center">EINSCHLÄGIGE DOKUMENTE</th><th>EP 88890182.4</th></tr>
</table>

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | US - A - 4 506 015 (HO et al.) <br><br> * Zusammenfassung; Spalte 4, Zeilen 23-37; Spalte 13, Zeilen 25-35 * <br><br> -- | 1,2 | C 12 N 11/14 <br><br> C 12 N 11/18 <br><br> A 23 L 2/34 |
| D,X | EP - A2 - 0 017 115 (BASF AKTIEN-GESELLSCHAFT) <br><br> * Anspruch 1; Seite 2, Zeile 32 - Seite 3, Zeile 12 * <br><br> -- | 1,2 | |
| D,A | GB - A - 1 368 854 (SNAM PROGETTI S.P.A.) <br><br> * Ansprüche 1,2 * <br><br> -- | 1,2 | |
| A | DE - A1 - 2 607 532 (RÖHM GMBH) <br><br> * Ansprüche 1,2 * <br><br> ---- | 1 | |

KLASSIFIKATION DER ANMELDUNG (Int. Cl.4):

C 12 N 11/14

C 12 N 11/18

A 23 L 2/34

RECHERCHIERTE SACHGEBIETE (Int. Cl.4)

C 12 N

A 23 L

Der vorliegende Recherchenbericht wurde für alle Patentansprüche erstellt.

| Recherchenort | Abschlußdatum der Recherche | Prüfer |
|---|---|---|
| WIEN | 12-10-1988 | WOLF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82